# EUROPÄISCHE PATENTSCHRIFT

(11) **EP 0 616 638 B1**
(45) Veröffentlichungstag und Bekanntmachung des Hinweises auf die Patenterteilung: **10.04.1996**
(21) Anmeldenummer: 92924636.1
(22) Anmeldetag: 01.12.1992
(51) Int. Cl.: C12M 1/12, B01D 39/00, C12N 15/10

(54) **VERFAHREN UND VORRICHTUNG ZUR ISOLIERUNG VON ZELLINHALTSSTOFFEN WIE NUKLEINSÄUREN AUS NATÜRLICHEN QUELLEN**
METHOD AND DEVICE FOR THE ISOLATION OF CELL COMPONENTS, SUCH AS NUCLEIC ACIDS, FROM NATURAL SOURCES
PROCEDE ET DISPOSITIF POUR L'ISOLATION D'EXTRAITS CELLULOSIQUES, TELS QU'ACIDES NUCLEIQUES PROVENANT DE SOURCES NATURELLES

(30) Priorität: 02.12.1991 DE 4139664
(43) Veröffentlichungstag der Anmeldung: 28.09.1994
(73) Patentinhaber: QIAGEN GmbH, D-40724 Hilden (DE)
(72) Erfinder: COLPAN, Metin, D-4300 Essen-Kettwig (DE)
(74) Vertreter: Meyers, Hans-Wilhelm, Dr.
(86) Internationale Anmeldenummer: EP9202774
(87) Internationale Veröffentlichungsnummer: WO9311218

(56) Entgegenhaltungen:
- EP-A- 0 431 905
- EP-A- 0 471 910
- WO-A-92/00132
- US-A- 2 114 748
- US-A- 4 935 142

## Beschreibung

Gegenstand der vorliegenden Erfindung ist ein Verfahren zur Isolierung von Inhaltsstoffen, wie Nukleinsäuren aus natürlichen Quellen durch Abtrennung der aufgeschlossenen natürlichen Quellen wie Zellen oder Zelltrümmer in einer Probe durch Filtration sowie eine Vorrichtung zur Durchführung des Verfahrens.

Bei der Präparation von Zellinhaltsstoffen, insbesondere von Nukleinsäuren, stellt sich oft das Problem, die aufgeschlossenen natürlichen Quellen, aus denen diese Inhaltsstoffe stammen, von den gelösten Stoffen zu trennen. Die Abtrennung der Zellen oder Zelltrümmer erfolgt mittels Zentrifugation, wobei sich die größeren Zelltrümmer oder Zellen als Pellet im Zentrifugationsröhrchen abscheiden. Die Zellinhaltsstoffe finden sich dann im Überstand und können pipettiert werden. An sich einfachere Filtrationsverfahren konnten sich insbesondere bei der Präparation von Nukleinsäuren nicht durchsetzen, da die aufgeschlossenen Zellen oder deren Bruchstücke entweder durch die zu grobporigen Filter durchlaufen und somit für Trübungen und Verunreinigungen im Filtrat sorgen oder bei Verwendung von Filtern mit entsprechenden engen Poren es jedoch zwangsläufig zur Verstopfung der Filter kommt, so daß eine sinnvolle Präparation der Zellinhaltsstoffe nicht mehr möglich ist.

Die WO 92/00132 beschreibt eine Filtrationseinrichtung mit beschichteten Filtermembranen und absoluten Porenfiltern, bei denen die Membranen und absoluten Porenfilter in zwei Bereichen der Filtereinrichtung angeordnet sind. Mit der Vorrichtung sollen wäßrige Pufferlösungen von DNA, Endotoxinen und Viren befreit werden.

Die EP-A-0 431 905 betrifft ein Verfahren zur Reinigung von Phagen-DNA, wobei eine Mischung enthaltend Mikroorganismen, Zellen und Phagen durch einen Membranfilter gegeben wird. Die Filterschicht des Membranfilters ist zur Sterilfiltration ausgelegt. Dabei passiert der Phage die Filtrationseinrichtung und kann dann durch nachfolgende Ultrafiltration weiter aufgearbeitet werden. Als Filterschicht dient dabei eine Membran, die eine Porengröße von 0,45 bis 0,22 µm aufweist.

Die US-PS 2,114,748 beschreibt poröse Filtrationseinrichtungen, die zwei Schichten von Glaspartikeln umfassen, wobei eine Schicht gröber ist und die andere Schicht eine feinere Körnung aufweist. Es handelt sich hierbei um eine Filtrationseinrichtung, die als erste Schicht, in Fließrichtung gesehen, die feinere Körnung aufweist, wohingegen die darunterliegende Schicht gröber strukturiert ist. Es handelt sich hierbei um eine Glasfritte.

US-PS 4,935,142 betrifft eine Vorrichtung, die zum Filtern und Trennen von Substanzen, insbesondere auf dem Gebiet der Membran-Affinitäts-Chromatographie, eingesetzt wird. Die Vorrichtung weist insbesondere eine Einlaß- und eine Auslaßöffnung auf, die einen Hohlkörper einschließen. Die Vorrichtung dient zur Aufnahme einer Filtrationseinrichtung, die aus einer mehrlagigen Filterschicht bestehen kann.

Der vorliegenden Erfindung liegt mithin das Problem zugrunde, ein Verfahren bereitzustellen und eine Vorrichtung zu schaffen, mit deren Hilfe Zentrifugationsschritte zur Präparation von Zellinhaltsstoffen aus natürlichen Quellen wie Zellen vermieden werden können durch einfacher zu handhabende Filtrationsschritte.

Das der Erfindung zugrundeliegende technische Problem wird gelöst durch ein Verfahren gemäß den Merkmalen des Anspruchs 1. Die sich daran anschließenden Unteransprüche betreffen bevorzugte Ausführungsformen des erfindungsgemäßen Verfahrens. Die erfindungsgemäße Vorrichtung weist die Merkmale des Anspruchs 10 auf. Die sich daran anschließenden Unteransprüche betreffen bevorzugte Ausführungsformen der erfindungsgemäßen Vorrichtung.

Um Inhaltsstoffe aus Zellen zu isolieren, werden diese überlicherweise zunächst aufgeschlossen. Bei der Präparation von Nukleinsäuren müssen die Zellen beispielsweise zunächst durch die Verwendung von Enzymen, wie z. B. Proteinase K, Lysozym und Detergenzien wie SDS, Brij, Triton X 100, Tween 20, DOC und Chemikalien wie Natriumhydroxid, Guanidin-Hydrochlorid und Guanidin-Isothiocyanat aufgeschlossen werden. Danach wird das so aufbereitete Probenmaterial einer Filtration unterworfen, wobei die Porengröße des zur Filtration zur Anwendung kommenden Filters in Fließrichtung der Probe abnimmt.

In einer bevorzugten Ausführungsform kann der Fluß der Probe bei der Filtration durch Anlegen eines Überdrucks oder Unterdrucks erleichtert werden. Durch die Konfiguration der Porengröße des Filters ist jedoch auch ein Durchgang der zu filtrierenden Probe durch den Filter, allein getrieben durch die Schwerkraft, möglich. Desweiteren kann auch, zur Beschleunigung des Passierens der Probe durch den Filter, die Probe durch Zentrifugation durch den Filter befördert werden.

Das erfindungsgemäße Verfahren ist insbesondere zur Präparation von Plasmid-DNA oder genomischer DNA mit einer Größe von 1 bis 50 Kb geeignet.

Als Filter, die im erfindungsgemäßen Verfahren eingesetzt werden können, kommen insbesondere solche aus gesintertem Polyethylen, Polypropylen, Polytetrafluoroethylen, Glas, Silicagel, Aluminiumoxid oder geschütteter Diatomeenerde, z. B. Cellit oder Silicagel, verwebten oder verklebten Vliesen aus Polypropylen, Polyester, Glasfasern und Silica sowie Papier, gepreßtem Papier, Vliesen aus Papier oder Kombinationen davon in Betracht.

In einer bevorzugten Ausführungsform des erfindungsgemäßen Verfahrens werden gleichzeitig mehrere Proben aufgearbeitet und durch entsprechende Vorrichtungen, die in vorteilhafter Weise an Mikrotitrationssysteme adaptiert sind, geführt.

Die erfindungsgemäße Vorrichtung zur Durchführung des Verfahrens besteht aus einem vorzugsweise zylindrischen Hohlkörper 40 mit Einlaß- und Auslaßöffnung 50, 60 sowie einer Filtrationseinrichtung 70, die im Hohlkörper 40 angeordnet ist, die aus einer mehrlagigen Filterschicht mit abnehmender Porengröße, in Richtung der Auslaßöffnung (60) gesehen, besteht, wobei die Filtrationseinrichtung (70) eine Porengröße im Bereich von 5 µm bis 500 µm aufweist, in einer Dicke der gesamten Filterschicht von 0,1 bis 10 mm. Zur Fixierung der Filtrationseinrichtung 70 können übliche Befestigungsmittel dienen wie beispielsweise Verklebungen oder aber auch Fixierung durch Reibungskräfte, indem die Filtrationseinrichtung 70 im Hohlkörper 40 eingeklemmt ist.

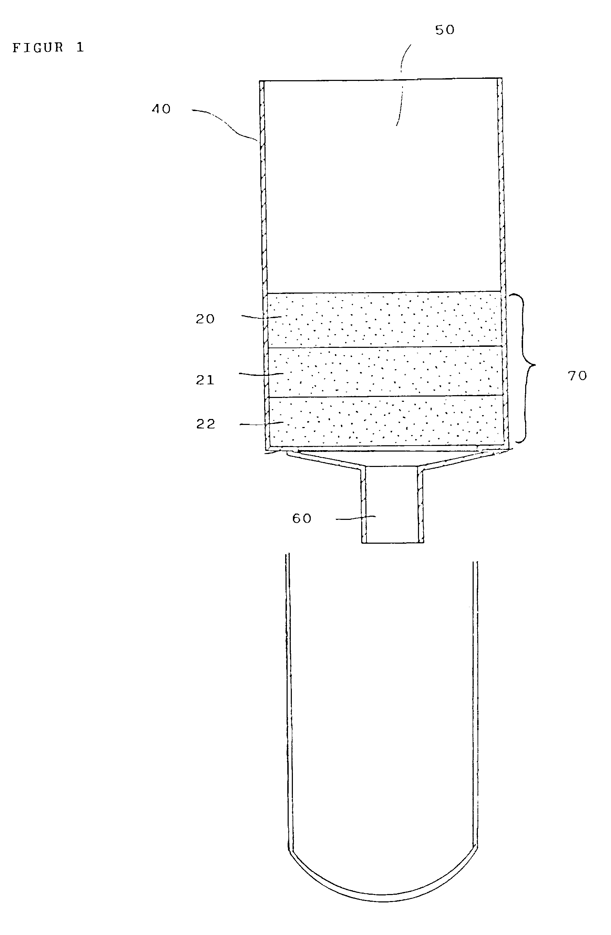

Die erfindungsgemäße Vorrichtung besteht aus mindestens einem Filter mit abnehmender Porengröße, in Richtung der Auslaßöffnung 60 gesehen. Die Figur 1 zeigt eine besonders bevorzugte Variante der erfindungsgemäßen Vorrichtung, indem im vorzugsweise zylindrischen Hohlkörper 40 die Filtrationseinrichtung 70 aus einer dreilagigen Schicht gestaltet ist. Die obere Schicht 20 kann dabei vorzugsweise Porengrößen von 100 bis 300 µm, die zweite Schicht eine Porengröße von 30 bis 100 µm und die dritte Schicht eine Porengröße von 5 bis 30 µm aufweisen. Als Materialien, aus denen die einzelnen Schichten bestehen können, kommen insbesondere solche aus gesintertem Polyethylen, Polypropylen, Polytetrafluoroethylen, Glas, Silicagel, Aluminiumoxid oder geschütteter Diatomeenerde, z. B. Cellit oder Silicagel, verwebten oder verklebten Vliesen aus Polypropylen, Polyester, Glasfasern und Silica sowie Papier, gepreßtem Papier, Vliesen aus Papier oder Kombinationen davon in Frage. Die Porengröße der Filterschicht liegt im Bereich von 5 µm bis 500 µm bei einer gesamten Dicke der Filterschicht von 0,1 bis 10 mm.

In einer besonders bevorzugten Ausführungsform kann beispielsweise die Filterschicht 20, 21 aus einem anorganischen Material mit der angegebenen Porengrößenabstufung bestehen, wohingegen die Filterschicht 22 auch aus Papier bestehen kann.

Es kann vorteilhaft sein, eine zusätzliche Schicht 23 in dem Hohlkörper 40 anzuordnen und zwar oberhalb der Schicht 20 oder unterhalb der Schicht 22, die ein vorzeitiges Eindringen der zu filtrierenden Lösung in den Filter oder das Austreten der Lösung aus der erfindungsgemäßen Vorrichtung verhindert. Es ist jedoch ebenso möglich, die Schicht 20 oder Schicht 22 als poröse, hydrophobe Schicht auszubilden. Ist die hydrophobe Trennschicht 23 oberhalb der Trennschicht 20 angeordnet, ist es vorteilhaft, wenn die Porengröße dieser Trennschicht nicht kleiner als diejenige der darunterliegenden Schicht 20 ist. Dieses Erfordernis ist bei der anderen Konfiguration, wobei die hydrophobe Trennschicht unterhalb der Schicht 20 angeordnet ist, nicht so kritisch.

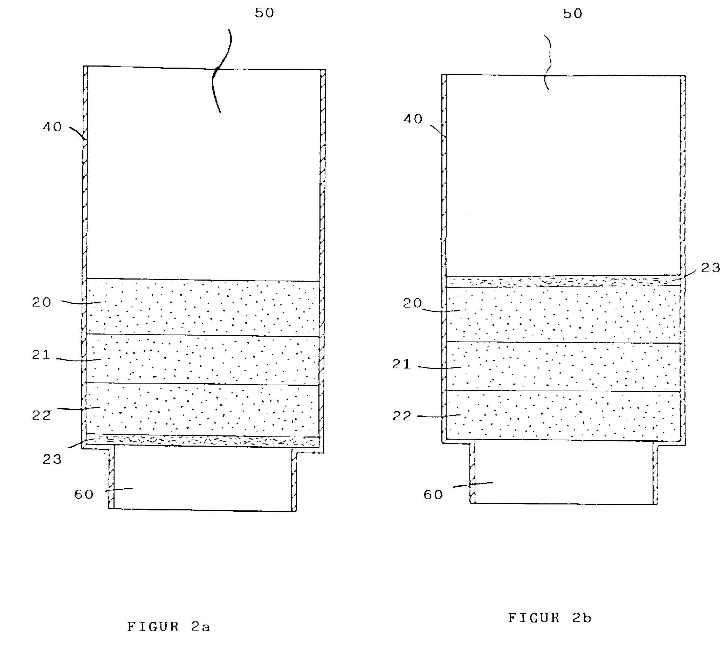

Die Figuren 2 a) und 2 b) zeigen die entsprechenden Konfigurationen mit hydrophober Trennschicht gemäß der bevorzugten Vorrichtung nach Figur 1.

In einer weiteren bevorzugten Ausführungsform ist die erfindungsgemäße Vorrichtung mit weiteren zur Nukleinsäurepräparation notwendigen Instrumenten kombinierbar und zwar solchen, wie sie in der parallelen Anmeldung P 41 39 664 näher beschrieben sind.

In der P 41 27 276 sind z. B. auch entsprechende Instrumente beschrieben. Der Anionenaustauscher ist in eine Membran eingebettet (3M Empore-Membran). Diese Systeme sind unter der Bezeichnung QIAwell handelsüblich.

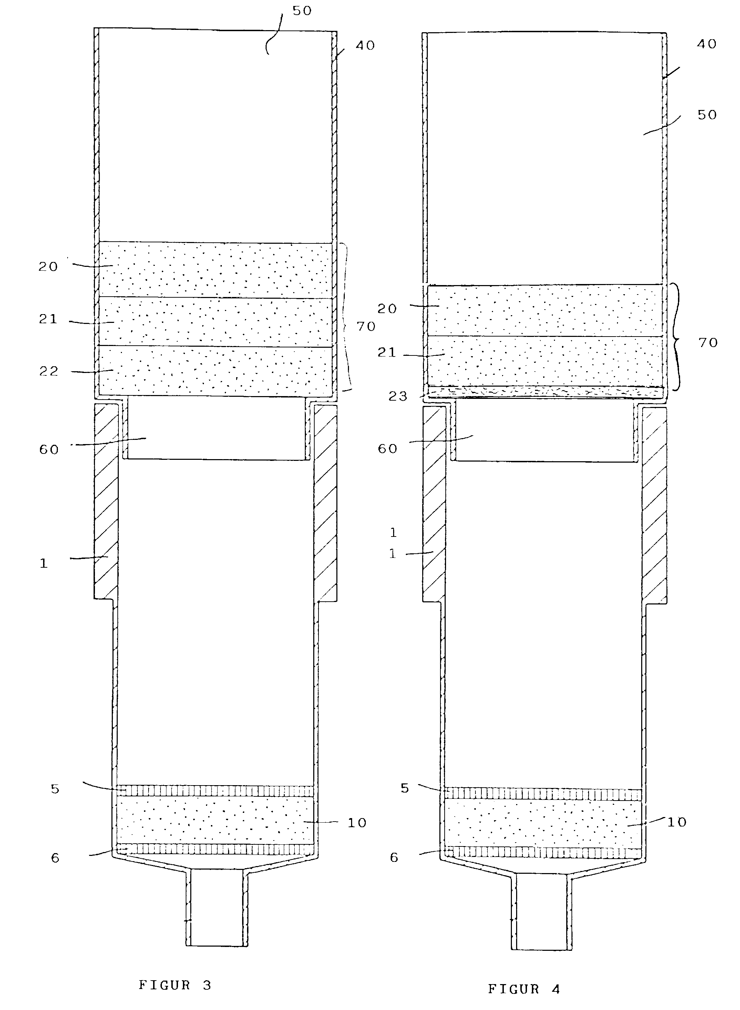

Die Figur 3 zeigt eine Anordnung, in dem die erfindungsgemäße Vorrichtung, bestehend aus einem dreilagigen Filter 20, 21, 22, auf eine Kartusche aufgesteckt ist, in der sich zwischen zwei Einrichtungen 5 und 6 ein Anionenaustauschermaterial 10 angeordnet befindet. Die aufzutrennende Probe wird auf die Filterschicht 20 geschichtet, der Filteraufsatz auf die Kartusche (im wesentlichen ein ebenfalls zylindrischer Hohlkörper) 1 (Extraktionssäule) aufgesteckt. Danach passiert die zu trennende Lösung die Filtereinrichtung 70, gegebenenfalls durch Anlegen eines leichten Über- oder Unterdruckes und tropft in den darunterliegenden Behälter (Extraktionssäule), in dem das Anionenaustauschermaterial 10 angeordnet ist. Unter geeigneten Pufferbedingungen (niedriger Salzkonzentration) binden dann beispielsweise Nukleinsäuren an dem Anionenaustauschermaterial. Die Zelltrümmer befinden sich in dem erfindungsgemäßen Filteraufsatz 40, wohingegen die interessierenden Nukleinsäuren an den Ionenaustauschern adsorbiert vorliegen. Nach Verwerfen des Filterkuchens in der Filtrationseinrichtung 40 kann dann der zylindrische Hohlkörper 1 mit den darin befindlichen Nukleinsäuren weiter verarbeitet werden.

Die Figur 4 zeigt eine analoge Situation wie Figur 3 mit dem erfindungsgemäßen Filteraufsatz 40, wobei ein zweilagiger Filter verwendet wird, der durch eine hydrophobe Trennschicht 23 abgeschlossen wird. Die Auslaßöffnung 60 der erfindungsgemäßen Vorrichtung wird vorzugsweise so dimensioniert, daß sie in die entsprechende Kartusche 1 des Nukleinsäure adsorbierenden Materials eingeschoben werden kann und dort aufgrund von Reibungskräften fixiert ist.

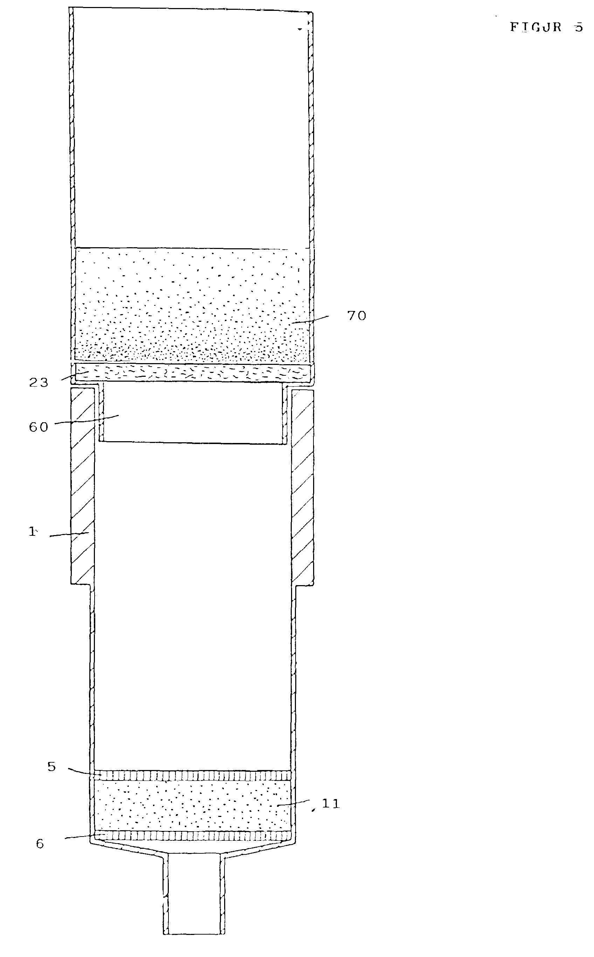

Die in den Figuren 1, 2 a) und 2 b) gezeigten Filtrationseinrichtungen können ebenfalls auf eine Kartusche aufgesteckt werden, in der ein Material angeordnet ist, das Nukleinsäuren bei hohen Ionenstärken zu binden vermag. Dies sind insbesondere Materialien wie Glas, Silicagel und andere mineralische Stoffe. Eine Nukleinsäurepräparation kann dann beispielsweise unter Hochsalzbedingungen durchgeführt werden. Die entsprechend vorbereitete und aufgeschlossene Probe wird dann durch die erfindungsgemäße Filtrationseinrichtung nach einer der Konfigurationen gemäß Figuren 1, 2a) oder 2b) passiert und dann in der Figur 5 gezeigten Vorrichtung 1 an Silicagel, Glasfaser, gepreßtem Glaspulver Materialien 11 unter Bedingungen hoher Ionenstärke adsorbiert.

Die Figur 5 zeigt die erfindungsgemäße Vorrichtung mit einer Filterschicht 70, die eine asymmetrische Porengrößenverteilung, in Fließrichtung der Probe gesehen, aufweist. Dabei nimmt in Fließrichtung die Porengröße kontinuierlich oder diskontinuierlich von etwa 100 bis 500 µm auf 5 bis 30 µm ab. Die Porengröße der Filterschicht variiert im Bereich von 5 µm bis 500 µm bei einer Dicke der Filterschicht von 0,1 bis 10 mm.

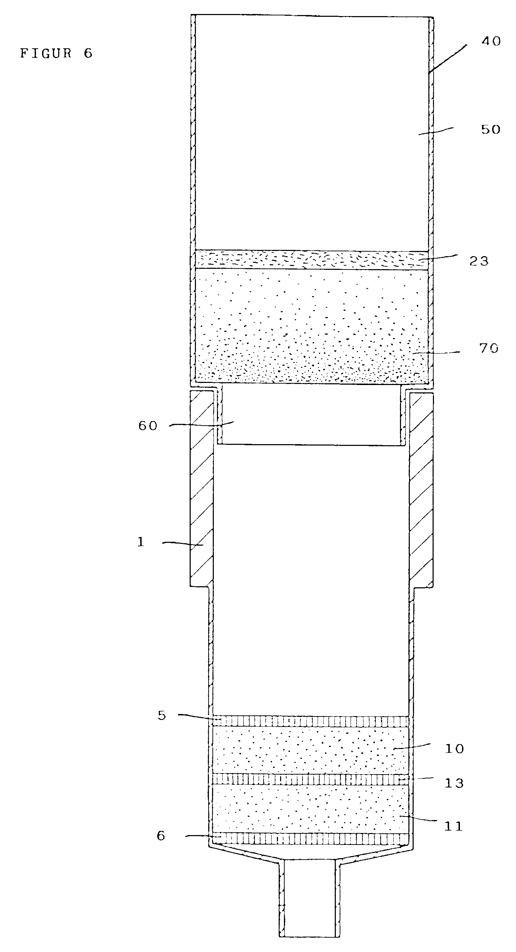

Die Figur 6 zeigt eine Anordnung, in der die erfindungsgemäße Vorrichtung in einer Konfiguration ähnlich der Figur 5 auf einer Extraktionssäule angeordnet ist, in der sich ein Anionenaustauschermaterial 10 oberhalb des mineralischen Trägers 11 gemäß Figur 5 angeordnet ist. Diese Anordnung ist vorteilhaft, da die aufgeschlossene Probe in der erfindungsgemäßen Vorrichtung von Zelltrümmern befreit wird und in die Extraktionssäule 1 hineintropft, um unter Niedrigsalzbedingungen an dem Anionenaustauschermaterial 10 adsorbiert zu werden. Nach dem Verwerfen des Filterkuchens kann dann die im Ionenaustauscher gebundene Nukleinsäure zunächst unter Bedingungen niedriger Ionenstärke gewaschen werden und dann unter Bedingungen hoher Ionenstärke vom Anionenaustauscher 10 eluiert werden. Daraufhin wird die unter Hochsalzbedingungen vom Anionenaustauscher eluierte Nukleinsäure vom anorganischen Material 11, beispielsweise Silicagel oder Glasfasern, adsorbiert. Nach weiteren Waschschritten kann dann durch Zugabe von beispielsweise destilliertem Wasser die RNA vom Träger 11 desorbiert und eluiert werden.

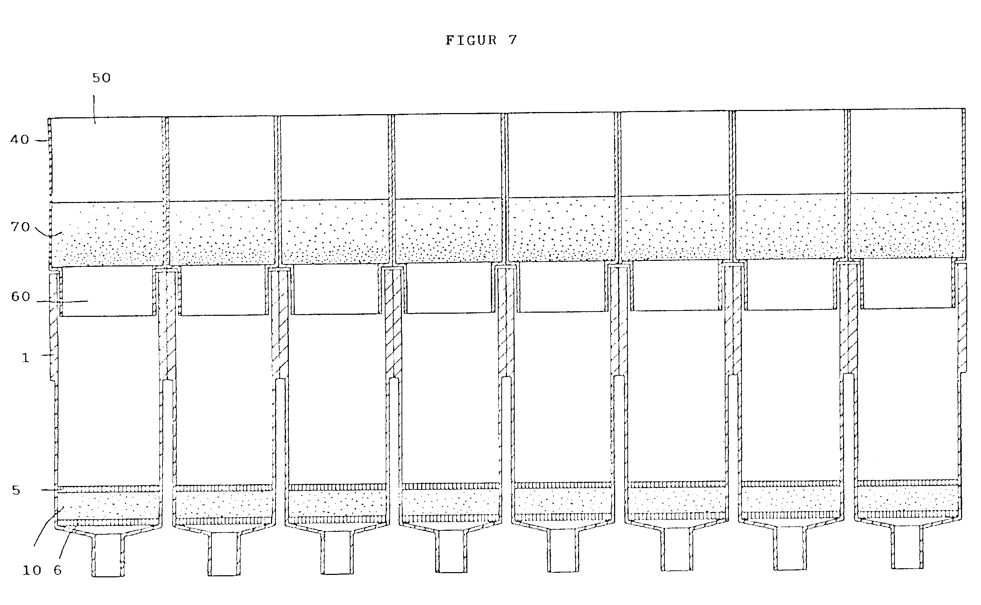

Die Figur 7 zeigt eine weitere bevorzugte Ausführungsform der erfindungsgemäßen Einrichtung, die in Form von Mikrotitrationsstreifen vorliegen kann. Die Figur 7 zeigt eine Anordnung, in der die erfindungsgemäßen Vorrichtungen 40, die linear hintereinander angeordnet sind und bereits auf eine entsprechende Anordnung von Kartuschen wie sie beispielsweise in Figur 3 bereits beschrieben wurde, angeordnet ist. Die hydrophobe Schicht 23 kann unter- oder oberhalb der Filtrationseinrichtung angeordnet sein.

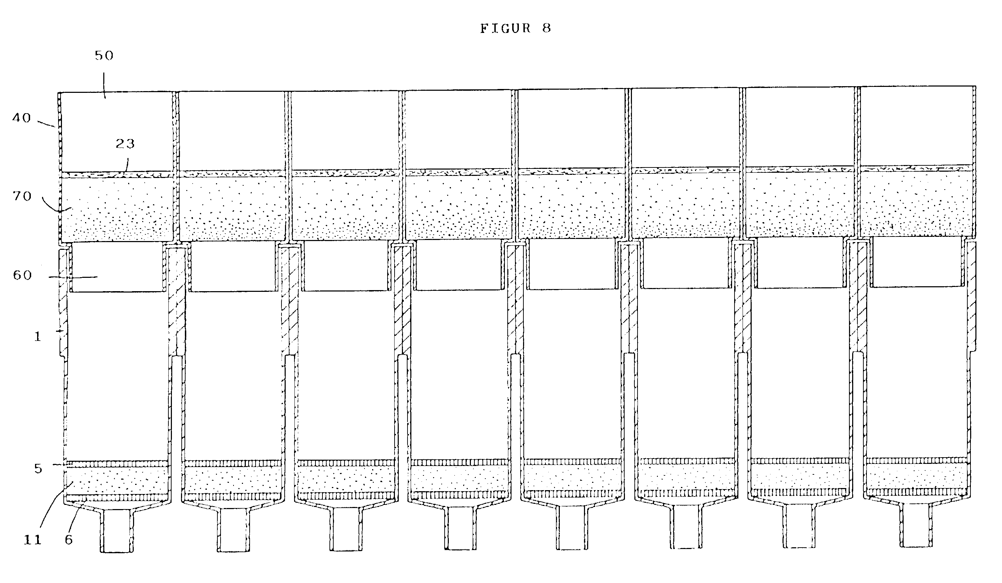

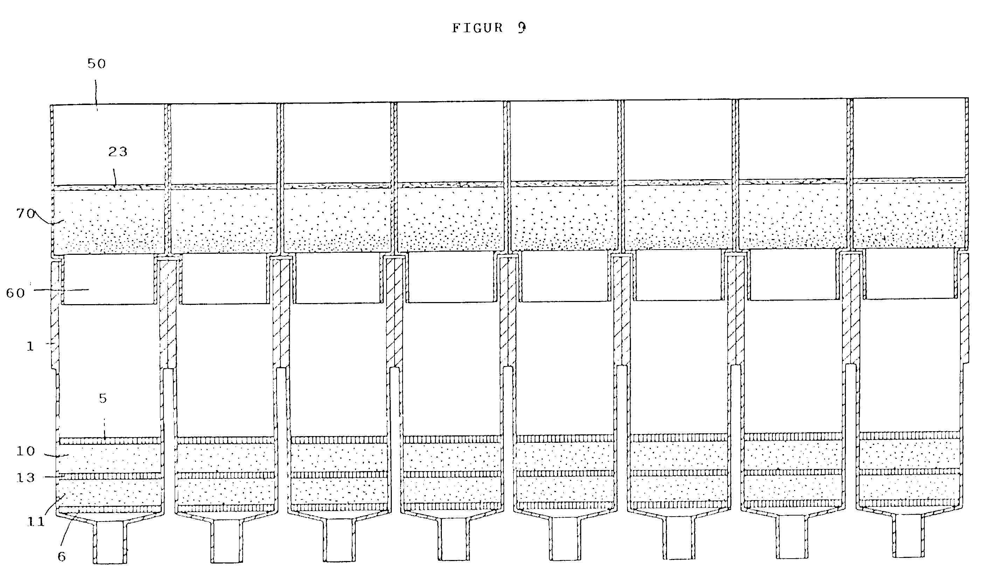

Entsprechende Konfigurationen sind mit jeweils anderen Adsorptionsmaterialien in den Figuren 8 und 9 beschrieben.

Das erfindungsgemäße Verfahren wird anhand der folgenden Beispiele näher erläuert.

### Beispiel 1

### Plasmid-Minipräparation

Eine 1,5 ml HB 101 E.coli Kultur mit pUC 18 Plasmid-DNA in LB-Medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert. Zur Zell-Lyse werden 0,6 ml 0,2 M NaOH, 1% SDS zur Zellsuspension gegeben, vorsichtig gemischt und 5 Minuten bei Raumtemperatur stehengelassen. Danach wird 0,25 ml 3 M K-Acetat, 2 M Essigsäure zur Neutralisation zugegeben, gemischt und 15 Minuten auf Eis inkubiert. Das Lysat wird in die Filtrationsvorrichtung nach Figur 1 überführt. Die ganze Vorrichtung wird auf eine Vakuumkammer aufgesetzt und das Zell-Lysat mit 20 mbar - 800 mbar Differenzdruck durch die erfindungsgemäße Filtervorrichtung in ein 2 ml Röhrchen gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die DNA im Filtrat wird mit 0,5 Volumen Iso-Propanol ausgefällt und das Alkoholpellet durch eine Zentrifugation pelletiert.

Wenn die erfindungsgemäße Filtrationsvorrichtung im Microtitrations-Format ausgelegt wird, können gleichzeitig bis zu 96 Proben aufgearbeitet werden. Somit läßt sich die Zeit für die Präparation von 96 Proben von 240 min auf 15 min reduzieren.

### Beispiel 2

### Plasmid-Minipräparation an Anionenaustauscher

Eine 1,5 ml HB 101 E.coli Kultur mit pUC 18 Plasmid-DNA in LB-Medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert. Zur Zell-Lyse werden 0,25 ml 0,2 M NaOH, 1% SDS zur Zellsuspension gegeben, vorsichtig gemischt und 5 Minuten bei Raumtemperatur stehengelassen. Danach wird 0,25 ml 3 M K-Acetat, 2 M Essigsäure zur Neutralisation zugegeben, gemischt und 15 Minuten auf Eis inkubiert. Das Lysat wird in die Filtrationsvorrichtung nach Figur 3. überführt. Die ganze Vorrichtung wird auf eine Vakuumkammer aufgesetzt und das Zell-Lysat mit 20 mbar - 800 mbar Differenzdruck durch die erfindungsgemäße Filtervorrichtung gesaugt und auf eine Anionenaustauschersäule filtriert. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Das Filtrat wird vollständig durch die Extraktionssäule gesaugt oder gedrückt, um eine Adsorption der DNA zu erreichen. Die Extraktionssäule wird anschließend 2 mal mit 1 ml 1 M NaCl, 15% Ethanol, 50 mM MOPS, pH 7,0 gewaschen, um RNA und Proteine zu entfernen. Die DNA wird mit 1 ml 1,25 M NaCl, 15% Ethanol, 50 mM Tris-HCl, pH 8,5 eluiert. Die eluierte DNA wird zur Entsalzung und zur Konzentrierung mit Alkohol gefällt und das Alkoholpellet durch eine Zentrifugation pelletiert.

### Beispiel 3

### Plasmid-Minipräparation mit Zell-Lyse im Filter

Eine 1,5 ml HB 101 E.coli Kultur mit pUC 18 Plasmid-DNA in LB-medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert und in die Filtrationsvorrichtung nach Figur 4 überführt. Zur Zell-Lyse werden 0,25 ml 0,2 M NaOH, 1% SDS zur Zellsuspension in die Filtrationsvorrichtung nach Fig. 4 gegeben, die Vorrichtung mit einem Stopfen oder einer Klebefolie verschlossen, vorsichtig gemischt und 5 Minuten bei Raumtemperatur stehengelassen. Danach wird 0,25 ml 3 M K-Acetat, 2 M Essigsäure zur Neutralisation zugegeben, die Vorrichtung mit einem Stopfen oder einer Klebefolie verschlossen, vorsichtig gemischt und 15 Minuten auf Eis Inkubiert. Durch die hydrophobe Trennschicht wird ein vorzeitiges Austreten der Lysereagenzien durch die Filterschicht vermieden, und eine Zell-Lyse direkt in der Filtereinheit ermöglicht. Die ganze Vorrichtung wird auf eine Vakuumkammer aufgesetzt und das Zell-Lysat mit 20 mbar - 800 mbar Differenzdruck durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird 2 mal mit 0,8 ml 1 M NaCl, 15% Ethanol, 50 MOPS, pH 7,0 gewaschen, um RNA und Proteine zu entfernen. Die DNA wird mit 1 ml 1,25 M Nacl, 15% Ethanol, 50 mM Tris-HCl, pH 8,5 eluiert. Die eluierte DNA wird zur Entsalzung und zur Konzentrierung mit Alkohol gefällt und das Alkoholpellet durch eine Zentrifugation pelletiert.

### Beispiel 4

### Plasmid-Minipräparation an Silicagel

Eine 1,5 ml HB 101 E. coli Kultur mit pUC 18 Plasmid-DNA in LB-Medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert. Zur Zell-Lyse weden 0,25 ml 0,2 M NaOH, 1% SDS zur Zellsuspension gegeben, vorsichtig gemischt und 5 Minuten bei Raumtemperatur stehengelassen. Danach wird 0,5 ml 4,5 M Guanidin-HCl, 0,75 M K-Acetat, pH 5,5 zur Neutralisation zugegeben, gemischt und 15 Minuten auf Eis inkubiert. Die ganze Vorrichtung nach Figur 5 wird auf eine Vakuumkammer aufgesetzt und das Zell-Lysat mit 20 mbar - 800 mbar durch die Vorrichtung in ein Probenröhrchen filtriert. Im Probenröhrchen befindet sich 10 mg eines mineralischen Trägers, z. B. Silicagel oder Glas mit einer Partikelgröße von 1 - 5 µm in 0,1 ml 4,5 M Guanidin-HCl, 0,75 M K-Acetat, pH 5,5, der in der Lage ist, die DNA unter diesen Bedingungen zu adsorbieren. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen.

Das Filtrat mit der DNA in Hochsalz und dem mineralischen Träger in Hochsalz wird 1 - 5 min inkubiert, um eine Adsorption der DNA zu erreichen. Die Suspension wird 5 min bei 3.000 rpm abzentrifugiert und der Überstand verworfen. Das Pellet wird in 1 ml 80% Ethanol, 10 mM Tris-HCl, pH 7,0 suspendiert und abermals abzentrifugiert, und das Pellet mit der gebundenen DNA 5 min bei Raumtemperatur an der Luft getrocknet. Zum Schluß wird die DNA mit 100 µl 10 mM Tris-HCl, pH 8,0 eluiert. Die eluierte DNA kann direkt in einer enzymatischen Reaktion wie z. B. Restriktionsspaltung, Markierung, Sequenzierung oder Amplifikation eingesetzt werden.

### Beispiel 5

### Plasmid-Minipräparation an Glasmembran

Eine 1,5 ml HB 101 E.coli Kultur mit pUC 18 Plasmid-DNA in LB-Medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert. Zur Zell-Lyse werden 0,25 ml 0,2 M NaOH, 1% SDS zur Zellsuspension gegeben, vorsichtig gemischt und 5 Minuten bei Raumtemperatur stehengelassen. Danach wird 0,5 ml 4,5 M Guanidin-HCl, 0,75 M K-Acetat, pH 5,5 zur Neutralisation zugegeben, gemischt und 15 Minuten auf Eis inkubiert. Die ganze Vorrichtung nach Figur 5 wird auf eine Vakuumkammer aufgesetzt und das Zell-Lysat mit 20 mbar - 800 mbar durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denatuierten Proteinen und dem ausgefallenen SDS verworfen. Das Filtrat mit der DNA in Hochsalz wird durch die Silicagelextraktionssäule mit einer z. B. 2 mm dicken Glasfasermembran gesaugt oder gedrückt um eine Adsorption der DNA zu erreichen. Die Extraktionssäule wird 2 mal mit 1 ml 6,5 M Guanidin-HCl, 10 mM Tris-HCl, pH 7,0 gewaschen, um RNA und Proteine zu entfernen. Die Extraktionssäule wird mit 2 mal 1 ml 80% Ethanol, 10 mM Tris-HCl, pH 7,0 und mit 1 mal 0,8 ml 90% Ethanol/Wasser gewaschen und die Ethanolspuren abgesaugt. Zum Schluß wird die DNA mit 10 µl 10 mM Tris-HCl, pH 8,0 eluiert und in neuen 1,5 ml Röhrchen aufgefangen. Die eluierte DNA kann direkt in einer enzymatischen Reaktion wie z. B. Restriktrionsspaltung, Markierung, Sequenzierung oder Amplifikation eingesetzt werden.

### Beispiel 6

### Plasmid-Minipräparation an Anionenaustauscher/Glasmembran

Eine 1,5 ml HB 101 E. coli Kultur mit pUC 18 Plasmid-DNA in LB-Medium wird 5 Minuten bei 3.000 g zentrifugiert, um die Zellen zu pelletieren. Das Zellpellet wird in 0,25 ml 50 ml Tris-HCl, 10 mM EDTA, pH 8,0, 100 µg/ml RNAse A resuspendiert und in eine Vorrichtung nach Figur 6 überführt. Zur Zell-Lyse werden 0,25 ml 0,2 M NaOH, 1% SDS zur Zellsuspension gegeben, vorsichtig gemischt und 5 Minuten bei Raumtemperatur stehengelassen. Danach wird 0,25 ml 3 M K-Acetat, 2 M Essigsäure zur Neutralisation zugegeben, die Vorrichtung mit einem Stopfen oder einer Klebefolie verschlossen, vorsichtig gemischt und 15 Minuten auf Eis inkubiert. Durch die hydrophobe Trennschicht wird ein vorzeitiges Austreten der Lysereagenzien durch die Filterschicht vermieden und eine Zell-Lyse direkt in der Filtereinheit ermöglicht.

Die ganze Vorrichtung wird auf eine Vakuumkammer aufgesetzt und das Zell-Lysat mit 20 mbar - 800 mbar Differenzdruck durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird mit 0,8 ml 1 M Nacl, 15% Ethanol, 50 mM MOPS, pH 7,0 und mit 0,8 ml 1 M NaClO₄ 15% Ethanol, 10 mM Na-Acetat pH 7,0 gewaschen, um RNA und Proteine zu entfernen. Die DNA wird mit 7 M NaClO₄, 5% Ethanol, 10 mM Tris-HCl, pH 7,0 von der Anionenaustauscherschicht 10 eluiert und dabei direkt an die Silicagelschicht 11 gebunden. Die Extraktionssäule wird mit 2 mal 0,8 ml 80% Ethanol, 100 mM NaCl, 10 mM Na-Acetat pH 7,0 und mit 0,8 ml 90% Ethanol/Wasser gewaschen. Die in der Extraktionsschicht vorhandenen Ethanol-H₂O-Reste werden durch ein Durchsaugen von Raumluft durch ein Vakuum für 1 - 2 Minuten verflüchtigt. Anschließend werden die Proben mit je 100 µl 1 mM Tris-HCl, pH 8,0 eluiert und in neuen 1,5 ml Röhrchen aufgefangen. Die eluierte DNA kann direkt in einer enzymatischen Reaktion wie z. B. Restriktionsspaltung, Markierung, Sequenzierung oder Amplifikation eingesetzt werden.

### Beispiel 7

### Plasmid-Minipräparation in einem "Mikrotitrationsstreifen"

8 mal 1,5 ml XL Blue E. coli Kulturen mit pUC 18 Plasmid-DNA in LB-Medium werden 5 Minuten bei 3.000 g zentrifugiert, um die Zellen zu pelletieren. Die Zellpellets werden in 0,25 ml 50 ml Tris-HCl, 10 mM EDTA, pH 8,0, 100 µg/ml RNAse A resuspendiert und in die Vorrichtung nach Figur 7 überführt. Zur Zell-Lyse werden 0,25 ml 0,2 M NaOH, 1% SDS zur Zellsuspension in die Filtrationsvorrichtung gegeben, die Vorrichtung mit einem Stopfen oder einer Klebefolie verschlossen, vorsichtig gemischt und 5 Minuten bei Raumtemperatur stehengelassen. Danach wird 0,25 ml 3 M K-Acetat, 2 M Essigsäure zur Neutralisation zugegeben, gemischt und 15 Minuten auf Eis inkubiert. Die ganze Vorrichtung wird auf eine Vakuumkammer aufgesetzt und das Zell-Lysat mit 20 mbar - 800 mbar durch die Vorrichtung gesaugt. Alternativ kann die Probe mit Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird mit 0,8 ml 1 M NaCl, 15% Ethanol, 50 mM MOPS, pH 7,0 gewaschen, um RNA und Proteine zu entfernen. Die DNA wird mit 8 mal 1 ml 1,25 M NaCl, 15% Ethanol, 50 mM Tris-HCl, pH 8,5 eluiert. Die eluierte DNA wird zur Entsalzung und zur Konzentrierung mit Alkohol gefällt und das Alkoholpellet durch eine Zentrifugation pelletiert.

### Beispiel 8

### Plasmid-Minipräparation in einem "Mikrotitrationsstreifen"

8 mal 1,5 ml LX Blue E.coli Kulturen mit pUC 18 Plasmid-DNA in LB-Medium werden 5 Minuten bei 3.000 g zentrifugiert, um die Zellen zu pelletieren. Die Zellpellets werden in 0,25 ml 50 ml Tris-HCl, 10 mM EDTA, pH 8,0, 100 µg/ml RNAse A resuspendiert und in die Vorrichtung nach Figur 8 überführt. Zur Zell-Lyse werden 0,25 ml 0,2 M NaOH, 1% SDS zur Zellsuspension in die Filtrationsvorrichtung gegeben, die Vorrichtung mit einem Stopfen oder einer Klebefolie verschlossen, vorsichtig gemischt und 5 Minuten bei Raumtemperatur stehengelassen. Danach wird 0,5 ml 4,5 M Guanidin-HCl, 0,75 M K-Acetat, pH 5,5 zur Neutralisation zugegeben, gemischt und 15 Minuten auf Eis inkubiert. Die ganze Vorrichtung nach Abb. G wird auf eine Vakuumkammer aufgesetzt und das Zell-Lysat mit 20 mbar - 800 mbar durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Das Filtrat mit der DNA in Hochsalz wird durch die Silicagelextraktionssäule mit einer 2 mm dicken Glasfasermembran gesaugt oder gedrückt um eine Adsorption der DNA zu erreichen. Die Extraktionssäule wird 1 mal mit 1 ml 6,5 M Guanidin-HCl, 10 mM Na-Acetat, pH 7,0 gewaschen, um RNA und Proteine zu entfernen. Die Extraktionssäule wird mit 2 mal 1 ml 80% Ethanol, 10 mM Tris-HCl, pH 7,0 und mit 0,8 ml 90% Ethanol/Wasser gewaschen und die Ethanolspuren durchgesaugt. Zum Schluß wird die DNA mit 100 µl 10 mM Tris-HCl, pH 8,0 eluiert und in neuen 1,5 ml Röhrchen aufgefangen. Die eluierte DNA kann direkt in einer enzymatischen Reaktion wie z. B. Restriktionsspaltung, Markierung, Sequenzierung oder Amplifikation eingesetzt werden.

### Beispiel 9

### Plasmid-Minipräparation in einem "Mikrotitrationsstreifen" mit Ionenaustauscher und Glasmembran

8 mal 1,5 ml XL Blue E. coli Kulturen mit pUC 18 Plasmid-DNA in LB-Medium werden 5 Minuten bei 3.000 g zentrifugiert, um die Zellen zu pelletieren. Die Zell-pellets werden in 0,25 ml 50 ml Tris-HCl, 10 mM EDTA, pH 8,0, 100 µg/ml RNAse A resuspendiert und in die Vorrichtung nach Figur 9 überführt. Zur Zell-Lyse werden 0,25 ml 0,2 M NaOH, 1% SDS zur Zellsuspension in die Filtrationsvorrichtung gegeben, die Vorrichtung mit einem Stopfen oder einer Klebefolie verschlossen, vorsichtig gemischt und 5 Minuten bei Raumtemperatur stehengelassen. Danach wird 0,25 ml 3 M K-Acetat 2 M Essigsäure zur Neutralisation zugegeben, gemischt und 15 Minuten auf Eis inkubiert. Die ganze Vorrichtung wird auf eine Vakuumkammer aufgesetzt und das Zell-Lysat mit 20 mbar - 800 mbar durch die Vorrichtung gesaugt. Alternativ kann die Probe mit Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird mit 0,8 ml 1 M NaCl, 15% Ethanol, 50 mM MOPS, pH 7,0 und mit 0,8 ml 1 M NaClO₄ 15% Ethanol, 10 mM Na-Acetat pH 7,0 gewaschen, um RNA und Proteien zu entfernen. Die DNA wird mit 7 M NaClO₄, 5% Ethanol, 10 mM Tris-HCl, pH 7,0 von der Anionenaustauscher-Schicht eluiert und dabei direkt an die Silicagelschicht gebunden. Die Extraktionssäule wird mit 8 mal 0,8ml 80% Ethanol, 100 mM NaCl, 10 mM Na-Acetat pH 7,0 und mit 8 mal 0,8 ml 90% Ethanol/Wasser gewaschen. Die in der Extraktionsschicht vorhandenen Ethanol-H₂O-Reste werden durch ein Durchsaugen von Raumluft durch ein Vakuum für 1 bis 2 Minuten verflüchtigt. Anschließend werden die 8 Proben mit je 10 µl 1 mM Tris-HCl, pH 8,0 eluiert.

### Beispiel 10

### Präparation von genomischer DNA

100 mg Lebergewebe von einer Ratte werden mit einem skalpell in 1 mm große Stücke zerkleinert und in 1 ml 500 mM Guanidin-HC1, 50 mM Tris-HC1, 10 mM EDTA, pH 8,0 suspendiert und die Zellen durch Zugabe von 0,1 ml Proteinase K (10 mg/ml) 2 Stunden bei 50°C lysiert. Das Lysat wird in die Filtrationsvorrichtung nach Figur 9 überführt. Die ganze Vorrichtung wird auf eine Vakuumkammer aufgesetzt und das Zell-Lysat mit 20 mbar - 800 mbar durch die Vorrichtung gesaugt. Alternativ kann die Probe mit Überdruck durch die Filtrationsschichten gedrückt werden. Nach der Filtration wird die Filtrationsvorrichtung abgenommen und der Filterkuchen mit den Zellbruchstücken und den denaturierten Proteinen verworfen. Die Extraktionssäule wird mit 0,8 ml 1 M NaCl, 15% Ethanol, 50 mM MOPS, pH 7,0 und mit 0,8 ml 1 M NaClO₄ 15% Ethanol, 10 mM Na-Acetat pH 7,0 gewaschen, um RNA und Proteine zu entfernen. Die DNA wird mit 7 M NaClO₄, 5% Ethanol, 10 mM Tris-HCl, pH 7,0 von der Anionenaustauscherschicht 10 eluiert und dabei direkt an die Silicagelschicht 11 gebunden. Die Extraktionssäule wird mit 8 mal 0,8 ml 80% Ethanol, 100 mM NaCl, 10 mM Na-Acetat pH 7,0 und mit 8 mal 0,8 ml 90% Ethanol/Wasser gewaschen. Die Probenröhrchen werden zur Entfernung der Hochsalzlösung mit 0,8 ml 70% Ethanol, 100 mM NaCl, 10 mM Na-Acetat, pH 7,0 und 0,8 ml 90% Ethanol/Wasser gewaschen. Die in der Extriktionsschicht vorhandenen Ethanol-H₂O-Reste werden durch ein Durchsaugen von Raumluft durch ein Vakuum für 1 - 2 Minuten verflüchtigt. Anschließend werden die 8 Proben mit je 50 µl 1 mM Tris-HCl, 0,1 mM EDTA, pH 8,0 eluiert.

### Beispiel 11

### Präparation von genomischer DNA mit Filtration und Glasmembran

100 mg Lebergewebe von einer Ratte werden mit einem Skalpell in 1 mm große Stücke zerkleinert und in 1 ml 500 mM Guanidin-HCl, 50 mM Tris-HCl, 10 mM EDTA, pH 8,0 suspendiert und die Zellen durch Zugabe von 0,1 ml Proteinase K (10 mg/ml) zwei Stunden bei 50°C lysiert. Danach wird 0,5 ml 5 M Guanidin-HCl, 10 mM Tris-HCl, pH 7 oder 0,25 ml Ethanol zugegeben und gemischt, um die Probe auf Adsorptionsbedingungen an das Glas einzustellen. Die ganze Vorrichtung nach Figur 9 wird auf eine Vakuumkammer aufgesetzt und das Zell-Lysat mit 20 mbar- 800 mbar durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken verworfen. Das Filtrat mit der DNA in Hochsalz oder in Ethanol wird durch die Silicagelextraktionssäule mit einer 2 mm dicken Glasfasermembran gesaugt oder gedrückt um eine Adsorption der DNA zu erreichen. Die Extraktionssäule wird 1 mal mit 1 ml 5 M Guanidin-HCl, 10 mM Tris-HCl, pH 7,0 gewaschen, um RNA und Proteine zu entfernen. Die Extraktionssäule wird mit 2 mal 1 ml 80% Ethanol, 10 mM Tris-HCl, pH 7,0 und mit 0,8 ml 90% Ethanol/Wasser gewaschen und die Ethanolspuren durchgesaugt. Zum Schluß wird die DNA mit 100 µl 10 mM Tris-HCl, pH 8,0 eluiert und in neuen 1,5 ml Röhrchen aufgefangen. Die eluierte genomische DNA kann direkt in einer enzymatischen Reaktion wie z. B. Restriktionsspaltung, Markierung oder Amplifizierung eingesetzt werden.

## Patentansprüche

1. Verfahren zur Isolierung von Zellinhaltsstoffen wie Nukleinsäuren aus natürlichen Quellen durch Abtrennung der aufgeschlossenen natürlichen Quellen, wie Zellen oder Zelltrümmer, in einer Probe mit Filtration, dadurch gekennzeichnet, daß die Probe einen Filter passiert, wobei die Porengröße des Filters in Fließrichtung der Probe durch den Filter abnimmt.

2. Verfahren nach Anspruch 1, wobei der Fluß der Probe durch den Filter durch Anlegen von Überdruck, Unterdruck oder Schwerkraft oder durch Zentrifugation erhöhte Schwerkraft sowie Kombination dieser Maßnahmen unterstützt wird.

3. Verfahren nach Anspruch 1 und/oder 2, wobei die Nukleinsäure Plasmid-DNA oder genomische DNA ist mit einer Größe von 1 bis 50 Kb (Kilobasenpaare).

4. Verfahren nach mindestens einem der Ansprüche 1 bis 3, dadurch gekennzeichnet, daß die Proben eine aus mehreren Schichten aufgebauten Filter passiert, wobei ausgehend von einer bestimmten Porengröße, die jeweils folgenden Schichten eine geringere Porengröße aufweisen.

5. Verfahren nach mindestens einem der Ansprüche 1 bis 4, dadurch gekennzeichnet, daß die Probe einen aus einer Schicht bestehenden Filter passiert, dessen Porengröße in Fließrichtung der Probe abnehmen.

6. Verfahren nach mindestens einem der Ansprüche 1 bis 5, dadurch gekennzeichnet, daß die Porengröße im Bereich von 5 µm bis 500 µm liegt, mit einer Dicke der gesamten Filterschicht von 0,1 mm bis 10 mm.

7. Verfahren nach mindestens einem der Ansprüche 1 bis 6, dadurch gekennzeichnet, daß die Probe eine dreilagige Filterschicht passiert, wobei die erste Filterschicht eine Porengröße von 100 bis 300 µm, die zweite Filterschicht eine Porengröße von 30 bis 100 µm und die dritte Filterschicht eine Porengröße von 5 bis 30 µm aufweist.

8. Verfahren nach mindestens einem der Ansprüche 1 bis 7, dadurch gekennzeichnet, daß die Filterschichten oder der Filter aus gesintertem Polyethylen, Polypropylen, Polytetrafluoroethylen, Glas, Silicagel, Aluminiumoxid oder geschütteten Diatomeenerde, z. B. Cellit oder Silicagel, verwebten oder verklebten Vliesen aus Polypropylen, Polyester, Glasfasern und Silica sowie Papier, gepreßtem Papier, Vliesen aus Papier aufgebaut sind.

9. Verfahren nach mindestens einem der Ansprüche 1 bis 8, wobei gleichzeitig mehrere Proben aufgearbeitet werden in Vorrichtungen, die an Mikrotitrationsverfahren adaptiert sind.

10. Vorrichtung zur Durchführung des Verfahrens nach einem der Ansprüche 1 bis 9, wobei in einem vorzugsweise zylindrischen Hohlkörper (40) mit Einlaß- und Auslaßöffnung (50, 60) eine Filtrationseinrichtung (70) angeordnet ist, die aus einer mehrlagigen Filterschicht mit abnehmender Porengröße, in Richtung der Auslaßöffnung (60) gesehen, besteht, wobei die Filtrationseinrichtung (70) eine Porengröße im Bereich von 5 µm bis 500 µm aufweist, in einer Dicke der gesamten Filterschicht von 0,1 bis 10 mm.

11. Vorrichtung nach Anspruch 10, dadurch gekennzeichnet, daß in dem zylindrischen Hohlkörper (40) eine hydrophobe Trennschicht (23) angeordnet ist.

12. Vorrichtung nach einem der Ansprüche 10 und/oder 11, dadurch gekennzeichnet, daß die Filtrationseinrichtung mit weiteren zur Präparation von Nukleinsäuren notwendigen Instrumenten, insbesondere zur Mikrotitration geeigneten Instrumenten, kombinierbar ist.

## Claims

1. A process for isolating cell ingredients, such as nucleic acids, from natural sources by separating the lysed natural sources, such as cells or cellular debris, in a sample by filtration, characterized in that said sample is passing a filter, the pore size of the filter decreasing in the direction of sample flow through the filter.

2. The process according to claim 1, wherein the sample flow through the filter is promoted by applying positive or negative pressure, or by gravity, or by gravity increased by centrifugation, or by a combination of said measures.

3. The process according to claim 1 and/or 2, wherein said nucleic acid is plasmid DNA or genomic DNA having a size of from 1 to 50 kb (kilo base pairs).

4. The process according to at least one of claims 1 to 3, characterized in that said sample is passing a filter composed of a multitude of layers wherein, with respect to a particular initial pore size, the subsequent layers have increasingly smaller pore sizes.

5. The process according to at least one of claims 1 to 4, characterized in that said sample is passing a filter composed of one layer whose pore size decreases in the direction of sample flow.

6. The process according to at least one of claims 1 to 5, characterized in that said pore size ranges from 5 µm to 500 µm, the total thickness of the filter bed being from 0.1 mm to 10 mm.

7. The process according to at least one of claims 1 to 6, characterized in that said sample is passing a three-layered filter bed wherein the first filter layer has a pore size of from 100 to 300 µm, the second filter layer has a pore size of from 30 to 100 µm, and the third filter layer has a pore size of from 5 to 30 µm.

8. The process according to at least one of claims 1 to 7, characterized in that said filter layers or said filter are composed of sintered polyethylene, polypropylene, polytetrafluorethylene, glass, silica gel, alumina, or packed diatomaceous earth, e.g. cellite or silica gel, interwoven or cemented nonwovens of polypropylene, polyester, glass fibers and silica, as well as paper, compressed paper, or paper non-wovens.

9. The process according to at least one of claims 1 to 8, wherein several samples are processed simultaneously in devices adapted to microtitration processes.

10. A device for performing the process according to one of claims 1 to 9, wherein in a preferably cylindrical hollow body (40) having an inlet and an outlet (50, 60), there is disposed filtration means (70) consisting of a multilayered filter bed having decreasing pore sizes as seen in the direction of outlet (60), the filtration means (70) having a pore size ranging from 5 µm to 500 µm, the total thickness of the filter bed being from 0.1 mm to 10 mm.

11. The device according to claim 10, characterized in that a hydrophobic separating layer (23) is disposed in said cylindrical hollow body (40).

12. The device according to one of claims 10 and/or 11, characterized in that said filtration means is capable of being combined with other instruments necessary for the preparation of nucleic acids, in particular with instruments suitable for microtitration.

## Revendications

1. Procédé pour l'isolement d'extraits cellulaires tels que les acides nucléiques provenant de sources naturelles, consistant à séparer par filtration les sources naturelles désagrégées telles que cellules ou débris de cellules présents dans un échantillon, caractérisé en ce que l'échantillon traverse un filtre dont la taille de pores diminue dans la direction d'écoulement de l'échantillon à travers le filtre.

2. Procédé selon la revendication 1, dans lequel l'écoulement de l'échantillon à travers le filtre est favorisé par application d'une surpression, d'une dépression ou de la force de gravité, ou de la force de gravité amplifiée par centrifugation, ainsi que par l'application d'une combinaison de ces mesures.

3. Procédé selon la revendication 1 et/ou 2, dans lequel l'acide nucléique est un ADN plasmide ou génomique d'une taille comprise entre 1 et 50 Kb (kilopaires de bases).

4. Procédé selon au moins l'une quelconque des revendications 1 à 3, caractérisé en ce que l'échantillon traverse un filtre, constitué de plusieurs couches, dans lequel, à partir d'une certaine taille de pores, les couches respectives suivantes présentent une taille de pores plus faible.

5. Procédé selon au moins l'une quelconque des revendications 1 à 4, caractérisé en ce que l'échantillon traverse un filtre composé d'une couche, dans lequel la taille de pores diminue dans la direction d'écoulement de l'échantillon.

6. Procédé selon au moins l'une quelconque des revendications 1 à 5, caractérisé en ce que la taille de pores est comprise entre 5 µm et 500 µm, l'épaisseur totale de la couche filtrante étant comprise entre 0,1 mm et 10 mm.

7. Procédé selon au moins l'une quelconque des revendications 1 à 6, caractérisé en ce que l'échantillon traverse un filtre à trois couches, dans lequel la première couche filtrante présente une taille de pores comprise entre 100 et 300 µm, la deuxième couche filtrante présente une taille de pores comprise entre 30 et 100 µm et la troisième couche filtrante présente une taille de pores comprise entre 5 et 30 µm.

8. Procédé selon au moins l'une quelconque des revendications 1 à 7, caractérisé en ce que les couches filtrantes ou le filtre sont composés de polyéthylène, de polypropylène, de polytetrafluoroéthylène, de verre, de gel de silice, d'oxyde d'aluminium frittés ou de terres de diatomées étalées, par exemple de cellite ou de gel de silice, de couches de fibres entrelacées ou collées de polypropylène, de polyester, de verre et de silice, ainsi que de papier, de papier comprimé, de fibres de papier.

9. Procédé selon au moins l'une quelconque des revendications 1 à 8, dans lequel plusieurs échantillons sont traités simultanément dans des dispositifs adaptés à un procédé de microtitration.

10. Dispositif pour la réalisation du procédé selon l'une quelconque des revendications 1 à 9, dans lequel un système de filtration (70) est disposé dans un corps creux (40), préférentiellement de forme cylindrique, avec des orifices d'entrée et de sortie (50, 60), lequel système est composé d'un filtre à plusieurs couches dont la taille de pores diminue dans la direction de l'orifice de sortie (60), le système de filtration (70) présentant une taille de pores comprise entre 5 µm et 500 µm, l'épaisseur totale de la couche filtrante étant comprise entre 0,1 et 10 mm.

11. Dispositif selon la revendication 10, caractérisé en ce que qu'une couche de séparation hydrophobe (23) est disposée dans le corps creux cylindrique (40).

12. Dispositif selon l'une quelconque des revendications 10 et/ou 11, caractérisé en ce que le système de filtration peut être combiné avec d'autres instruments nécessaires pour la préparation d'acides nucléiques, en particulier avec des instruments appropriés à la microtitration.
